# EUROPÄISCHE PATENTSCHRIFT

(11) **EP 1 620 415 B1**
(45) Veröffentlichungstag und Bekanntmachung des Hinweises auf die Patenterteilung: **21.11.2007**
(21) Anmeldenummer: 04727858.5
(22) Anmeldetag: 16.04.2004
(51) Int. Cl.: C07D 301/12

(54) **VERFAHREN ZUR HERSTELLUNG EINES ALKENOXIDS**
METHOD FOR PRODUCING AN ALKENE OXIDE
PROCEDE DE PRODUCTION D'UN OXYDE D'ALCENE

(30) Priorität: 16.04.2003 DE 10317520
(43) Veröffentlichungstag der Anmeldung: 01.02.2006
(73) Patentinhaber: BASF Aktiengesellschaft, 67056 Ludwigshafen (DE)
(72) Erfinder: GÖBBEL, Hans-Georg, 67169 Kallstadt (DE); BASSLER, Peter, 68519 Viernheim (DE); TELES, Joaquim, Henrique, 67166 Otterstadt (DE); RUDOLF, Peter, 68526 Ladenburg (DE)
(74) Vertreter: Isenbruck, Günter
(86) Internationale Anmeldenummer: PCT/EP2004/004077
(87) Internationale Veröffentlichungsnummer: WO 2004/092149

(56) Entgegenhaltungen:
- EP-A- 0 573 887
- EP-A- 0 659 473
- EP-A- 1 247 805
- EP-A- 1 285 915
- WO-A-00/07965

## Beschreibung

Die vorliegende Erfindung betrifft ein Verfahren zur Herstellung eines Alkenoxids, in dessen Verlauf ein Strom aus einem komprimierten, flüssigen Alken unter Verdampfung mindestens eines Teils des Alkens entspannt wird und gemäß einer bevorzugten Ausführungsform der Erfindung die durch die Entspannung aufgenommene Wärme verwendet wird, im erfindungsgemäßen Verfahren in mindestens einem Schritt Kälteleistung zur Verfügung zu stellen und somit eine Wärmeintegration im Gesamtverfahren zu gewährleisten.

Bei Verfahren zur Herstellung von Alkenoxiden wie beispielsweise Verfahren zur Herstellung von Propylenoxid aus Propen wird häufig ein Alkenstrom zur Umsetzung in das Verfahren eingeführt, der im großtechnischen Maßstab in flüssiger Form unter einem gewissen Druck über Rohrleitungen bezogen wird. Im Regelfall wird das flüssige Alken beispielsweise vor der Umsetzung zu dem entsprechenden Alkenoxid in ein Lösungsmittel eingemischt, das für die Umsetzung eingesetzt wird.

Eine Aufgabe, die der vorliegenden Erfindung zugrunde lag, war es, ein Verfahren bereitzustellen, das die in dem komprimierten Alkenstrom enthaltene Energie zumindest teilweise nutzt.

Demgemäß betrifft die vorliegende Erfindung ein Verfahren zur Herstellung eines Alkenoxids, umfassend mindestens die Schritte (i), (ii) und (v):
(i) Bereitstellen eines Stroms S1, enthaltend komprimiertes, flüssiges Alken;
(ii) Entspannen mindestens eines Teils des Stroms S 1 unter Aufnahme von Wärme und unter mindestens teilweisem Verdampfen des flüssigen Alkens;
(v) Umsetzung des gemäß (ii) erhaltenen Alkens mit einem Hydroperoxid in Anwesenheit mindestens eines Lösungsmittels und mindestens eines Katalysators unter Erhalt eines Gemischs, enthaltend Alkenoxid und das mindestens eine Lösungsmittel.

Im Rahmen der vorliegenden Erfindung kann hierbei gemäß (i) jedes Alken eingesetzt werden, das zu einem flüssigen Strom S1 komprimiert und mit einem Hydroperoxid zu dem entsprechenden Alkenoxid umgesetzt werden kann. Unter dem Begriff "Alken", wie er im Rahmen der vorliegenden Erfindung verwendet wird, werden sämtliche Verbindungen verstanden, die mindestens eine C-C-Doppelbindung aufweisen. Insbesondere sind im erfindungsgemäßen Verfahren beispielsweise folgende Alkene einsetzbar:
Ethen, Propen, 1-Buten, 2-Buten, Isobuten, Butadien, Pentene, Piperylen, Hexene, Hexadiene, Methylencyclopropan, Cyclopenten, Cyclohexen, Vinyloxiran, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Ethoxyethen, ungesättigte Carbonsäuren wie z.B. Acrylsäure, Methacrylsäure, Crotonsäure, Maleinsäure, Vinylessigsäure und Gemische aus zwei oder mehr dieser Verbindungen.

Bevorzugt werden im erfindungsgemäßen Verfahren Alkene verwendet, die 2 bis 6 Kohlenstoffatome enthalten. Insbesondere bevorzugt wird im Rahmen des erfindungsgemäßen Verfahrens als Alken Propen eingesetzt.

Demgemäß beschreibt die vorliegende Erfindung ein Verfahren, wie oben beschrieben, das dadurch gekennzeichnet ist, dass das im Strom S1 enthaltene Alken Propen ist.

Im erfindungsgemäßen Verfahren wird zur Umsetzung des Alkens mindestens ein Hydroperoxid eingesetzt. Im Rahmen der vorliegenden Erfindung wird unter dem Begriff "Hydroperoxid" eine Verbindung der allgemeinen Formel ROOH verstanden. Details bezüglich der Hydroperoxidherstellung und von im Rahmen des erfindungsgemäßen Verfahrens unter anderem einsetzbaren Hydroperoxiden sind in der DE-A 198 35 907 zu entnehmen, deren diesbezüglicher Inhalt in den Kontext der vorliegenden Anmeldung aufgenommen wird. Beispiele für erfindungsgemäß einsetzbare Hydroperoxide sind unter anderem tert-Butylhydroperoxid, Ethylbenzolhydroperoxid, tert-Amylhydroperoxid, Cumenhydroperoxid, Cyclohexylhydroperoxid, Methylcyclohexylhydroperoxid, Tetrahydronaphthalinhydroperoxid, Isobutylbenzolhydroperoxid, Ethylnaphthalinhydroperoxid, Persäuren wie beispielsweise Peressigsäure oder Wasserstoffperoxid. Auch Gemische aus zwei oder mehr Hydroperoxiden sind erfindungsgemäß einsetzbar. Bevorzugt wird im Rahmen der vorliegenden Erfindung Wasserstoffperoxid als Hydroperoxid eingesetzt, wobei weiter bevorzugt eine wässrige Wasserstoffperoxidlösung eingesetzt wird.

Demgemäß beschreibt die vorliegende Erfindung ein Verfahren, wie oben beschrieben, das dadurch gekennzeichnet ist, dass das gemäß (v) eingesetzte Hydroperoxid Wasserstoffperoxid ist.

Bezüglich der im Rahmen der vorliegenden Erfindung einsetzbaren Katalysatoren existieren keine besonderen Beschränkungen, solange in Anwesenheit der Katalysatoren das eingesetzte Alken zu dem entsprechenden Alkenoxid umgesetzt werden kann. Besonders bevorzugt werden im erfindungsgemäßen Verfahren Zeolith-Katalysatoren eingesetzt.

Zeolithe sind bekanntermaßen kristalline Alumosilikate mit geordneten Kanal- und Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolithe Structure Types", Elsevier, 5. Auflage, Amsterdam 2001.

Es sind nun auch Zeolithe bekannt, die kein Aluminium enthalten und bei denen im Silikatgitter an Stelle des Si(IV) teilweise Titan als Ti(IV) steht. Diese Titanzeolithe, insbesondere solche mit einer Kristallstruktur von MFI-Typ, sowie Möglichkeiten zu Ihrer Herstellung sind beispielsweise in der EP-A 0 311 983 oder der EP-A 0 405 978 beschrieben. Außer Silicium und Titan können solche Materialien auch zusätzliche Elemente wie z. B. Aluminium, Zirkonium, Zinn, Eisen, Kobalt, Nickel, Gallium, Germanium, Bor oder geringe Menge an Fluor enthalten. In den mit dem erfindungsgemäßen Verfahren vorzugsweise regenerierten Zeolith-Katalysatoren kann das Titan des Zeolithen teilweise oder vollständig durch Vanadium, Zirkonium, Chrom oder Niob oder einem Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0, 1 : 1.

Titanzeolithe, insbesondere solche mit einer Kristallstruktur vom MFI-Typ, sowie Möglichkeiten zu ihrer Herstellung sind beispielsweise in der WO 98/55228, EP-A 0 311 983 oder der EP-A 0 405 978 beschrieben, deren diesbezüglicher Umfang vollumfänglich in den Kontext der vorliegenden Anmeldung einbezogen wird.

Titanzeolithe mit MFI-Struktur sind dafür bekannt, dass sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan, Germanium, Tellur, Vanadium, Chrom, Niob, und Zirkonium enthaltende Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI , CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Bevorzugt werden im Rahmen der vorliegenden Erfindung Ti-Zeolithe mit MFI-, MEL- oder MFI/MEL-Mischstruktur eingesetzt. Als weiterhin bevorzugt sind im einzelnen die Tienthaltenden Zeolith-Katalysatoren, die im allgemeinen als "TS-1", "TS-2", "TS-3" bezeichnet werden, sowie Ti-Zeolithe mit einer zu beta-Zeolith isomorphen Gerüststruktur zu nennen. Ganz besonders bevorzugt ist im Rahmen der vorliegenden Erfindung ein ZeolithKatalysator vom TS-1-Typ.

Demgemäß beschreibt die vorliegende Erfindung ein Verfahren, wie oben beschrieben, das dadurch gekennzeichnet ist, dass der gemäß (v) eingesetzte Katalysator ein Titansilikalit-Katalysator vom TS-1-Typ ist.

Für die im Rahmen des erfindungsgemäßen Verfahrens einsetzbaren Lösungsmittel existieren im wesentlichen keine Beschränkungen, solange gewährleistet ist, dass in Anwesenheit dieses mindestens einen Lösungsmittels die Umsetzung des eingesetzten Alkens zu dem entsprechenden Alkenoxid in Anwesenheit des oben genannten Katalysators mit den oben beschriebenen Hydroperoxiden durchgeführt werden kann. Unter anderem bevorzugt sind beispielsweise
- Wasser,
- Alkohole, bevorzugt niedere Alkohole, weiter bevorzugt Alkohole mit weniger als 6 Kohlenstoffatomen wie beispielsweise Methanol, Ethanol, Propanole, Butanole und Pentanole,
- Diole oder Polyole, bevorzugt solche mit weniger als 6 Kohlenstoffatomen,
- Ether, wie beispielsweise Diethylether, Tetrahydrofuran, Dioxan, 1,2-Dietoxymethan, 2-Methoxyethanol,
- Ester, wie beispielsweise Methylacetat oder Butyrolacton,
- Amide, wie beispielsweise Dimethylformamid, Dimethylacetamid, N-Methylpyrrolidon,
- Ketone, wie beispielsweise Aceton,
- Nitrile, wie beispielsweise Acetonitril,
- oder Gemische aus zwei oder mehr der vorgenannten Verbindungen.

Im Rahmen einer bevorzugten Ausführungsform des erfindungsgemäßen Verfahrens wird als Lösungsmittel Methanol eingesetzt, wobei zusätzlich zu Methanol beispielsweise eines oder mehrere der oben genannten Lösungsmittel eingesetzt werden können. Unter anderem bevorzugt sei etwa Wasser genannt.

Demgemäß beschreibt die vorliegende Erfindung ein Verfahren, wie oben beschrieben, das dadurch gekennzeichnet ist, dass das gemäß (v) eingesetzte mindestens eine Lösungsmittel Methanol ist.

Gemäß der oben beschriebenen bevorzugten Ausführungsformen des erfindungsgemäßen Verfahrens betrifft die vorliegende Erfindung somit auch ein Verfahren, wie oben beschrieben, das dadurch gekennzeichnet ist, dass das Alken Propen, das Hydroperoxid Wasserstoffperoxid, der verwendete Katalysator ein Titansilikalit-Katalysator und das Lösungsmittel Methanol ist.

Der gemäß (i) bereitgestellte Strom S1 kann das Alken unter einem beliebigen Druck enthalten, solange gewährleistet ist, dass der Strom S1 hierbei unter den zusätzlich gewählten Temperaturbedingungen flüssig ist. Weiterhin hängt der Druckbereich des komprimierten Stroms S1 von dem verwendeten Alken oder Alkengemisch ab. Im Falle des wie oben beschrieben besonders bevorzugt eingesetzten Propens enthält gemäß einer bevorzugten Ausführungsform der Strom S1 das Propen unter einem Druck im Bereich von 20 bis 35 bar. Die Temperaturen des Stroms S1 liegen hierbei bevorzugt in einem Bereich von 5 bis 30 °C, bevorzugt in einem Bereich von 10 bis 30 °C, weiter bevorzugt in einem Bereich von 15 bis 30 °C und besonders bevorzugt in einem Bereich von 20 bis 30 °C.

Demgemäß betrifft die vorliegende Erfindung auch ein Verfahren, wie oben beschrieben, das dadurch gekennzeichnet ist, dass der Strom S1 gemäß (i) flüssiges Propen bei einem Druck im Bereich von 20 bis 35 bar und einer Temperatur im Bereich von 5 bis 30 °C enthält.

Der Strom S1, enthaltend flüssiges Propen, kann in einer bevorzugten Ausführungsform bis zu 0,5 Gew.-%, weiter bevorzugt bis zu 1 Gew.-%, besonders bevorzugt bis zu 2 Gew.-% und insbesondere bevorzugt bis zu 4 Gew.-% Propan enthalten.

Erfindungsgemäß wird mindestens ein Teil des komprimierten Alkenstroms S1 gemäß Schritt (ii) unter Aufnahme von Wärme entspannt, wobei mindestens ein Teil des flüssigen Alkens verdampft. Besonders bevorzugt sind im Rahmen des erfindungsgemäßen Verfahrens Verfahrensführungen, bei denen der gesamte Strom S1, der das umzusetzende mindestens eine Alken enthält, entspannt wird und dabei weiter bevorzugt das flüssige Alken vollständig beim Entspannen verdampft wird.

Der zu entspannende Strom S1 kann hierbei gemäß sämtlicher geeigneter Methoden und unter Verwendung sämtlicher geeigneter Vorrichtungen entspannt werden. Unter anderem seien etwa Ventile, Hähne, Blenden oder Drosseln genannt, die zum Entspannen eingesetzt werden können. Der Entspannungsvorgang kann hierbei in einem oder auch mehreren Schritten durchgeführt werden, wobei beispielsweise für die einzelnen Entspannungsschritte jeweils die gleiche oder mindestens zwei verschiedene Vorrichtungen verwendet werden können. Im Rahmen der vorliegenden Erfindung werden diesbezüglich unter dem Begriff "verschiedene Vorrichtungen" auch Vorrichtungen verstanden, die bis auf ihre Dimensionierung gleich sind. Unterschiedliche Vorrichtungen in diesem Sinne sind beispielsweise ein Ventil und ein Hahn oder zwei Ventile unterschiedlicher Bauart oder zwei Ventile gleicher Bauart mit unterschiedlichen Größen.

Der Druck, unter dem das gasförmige Alken nach dem Entspannen steht, kann im Wesentlichen beliebig gewählt werden und ist insbesondere von der Temperatur des zur vollständigen Verdampfung herangezogenen Heizmediums abhängig. In besonders bevorzugten Ausführungsformen wird der Druck, unter dem das entspannte Alken steht, so gewählt, dass durch den Entspannungs- und Verdampfungsprozess eine ausreichende Kälteleistung bei einer Temperatur im Bereich von -10 bis +20 °C erzeugt wird.

Beispielsweise im Fall des besonders bevorzugt eingesetzten Propens werden Drücke des entspannten Propens gewählt, die bevorzugt im Bereich von 4 bis 10 bar, bevorzugt im Bereich von 5 bis 9 bar und besonders bevorzugt im Bereich von 5 bis 8 bar liegen.

Demgemäß betrifft die vorliegende Erfindung auch ein Verfahren, wie oben beschrieben, das dadurch gekennzeichnet ist, dass der mindestens eine Teil des Stroms S 1 auf einen Druck im Bereich von 4 bis 10 bar entspannt wird.

Gemäß einer ganz besonders bevorzugten Ausführungsform wird der Druck, auf den das Alken entspannt wird, so gewählt, dass beim Entspannen des komprimierten Stroms eine nutzbare Kälteleistung erzeugt wird.

Unter dem Begriff "nutzbare Kälteleistung", wie er im Rahmen der vorliegenden Erfindung verwendet wird, wird eine Kälteleistung verstanden, die in mindestens einem anderen als dem erfindungsgemäßen Verfahren oder dem erfindungsgemäßen Verfahren selbst oder sowohl in mindestens einem anderen als dem erfindungsgemäßen Verfahren als auch dem erfindungsgemäßen Verfahren selbst zu Kühlzwecken eingesetzt werden kann.

Die Kühlung kann dabei beispielsweise derart erfolgen, dass beim Entspannen des komprimierten Stroms S 1 die dazu erforderliche Wärmemenge mindestens einem Kältemittel entzogen wird. Das derart abgekühlte, mindestens eine Kältemittel kann dann im Weiteren im erfindungsgemäßen Verfahren selbst oder in einem anderen als dem erfindungsgemäßen Verfahren zu Kühlzwecken eingesetzt werden.

Demgemäß beschreibt die vorliegende Erfindung auch ein Verfahren, wie oben beschrieben, das dadurch gekennzeichnet ist, dass die gemäß (ii) erzeugte Kälteleistung mindestens einem Kältemittel zugeführt wird.

Neben sämtlichen weiteren denkbaren Kältemitteln wird im erfindungsgemäßen Verfahren beispielsweise bevorzugt ein Propylenglykol-Wasser-Gemisch als Kältemittel eingesetzt.

Bevorzugt erfolgt im Rahmen der vorliegenden Erfindung die Entspannung des unter Druck stehenden Alkenstroms S 1 in mindestens einen Wärmetauscher hinein. In diesem Zusammenhang kann der Strom S 1 beispielsweise in einen einzigen Wärmetauscher hinein entspannt werden. Ebenso ist es möglich, den Strom S 1 beispielsweise in einen ersten Wärmetauscher unter Verwendung mindestens einer der oben genannten Entspannungsvorrichtungen wie beispielsweise Ventil, Hahn, Blende oder Drossel hinein auf einen bestimmten Druck zu entspannen und den in diesem ersten Schritt entspannten Strom S 1 in mindestens einen weiteren Wärmetauscher unter Verwendung mindestens einer weiteren der oben genannten Entspannungsvorrichtungen pro Wärmetauscher hinein weiter zu entspannen. Hierbei ist es demgemäß möglich, den Strom S 1 zunächst in dem ersten Wärmetauscher teilweise und in dem zweiten Wärmetauscher vollständig zu verdampfen.

In jedem der verwendeten Wärmetauscher kann die zur Verdampfung erforderliche Wärme beispielsweise mindestens einem Kältemittel entzogen werden, wobei in jedem Wärmetauscher ein einziges oder auch mehrere verschiedene Kältemittel eingesetzt werden können und die in den einzelnen Wärmetauschern eingesetzten Kältemittel oder Kältemittelgemische gleich oder voneinander verschieden sein können.

Bevorzugt wird im erfindungsgemäßen Verfahren der zu entspannende Strom S 1 in einen einzigen Wärmetauscher hinein entspannt, wobei als Entspannungsvorrichtung eine Blende oder ein Ventil verwendet wird.

Ebenso umfasst die vorliegende Erfindung Ausführungsformen, bei denen der Strom S 1 in zwei oder mehr Teilströme geteilt wird und jeder dieser Teilströme in jeweils einen Wärmetauscher hinein entspannt wird. Mit dieser ebenfalls bevorzugten Ausführungsform können, je nach Aufteilung des Stroms S1, in den einzelnen Wärmetauschern durch vollständige Verdampfung des jeweiligen Teilstroms gleiche oder unterschiedliche Kälteleistungen erzeugt werden.

Der im erfindungsgemäßen Verfahren zur Entspannung des Stroms S1 eingesetzte mindestens eine Wärmetauscher kann hierbei sämtliche geeignete Ausführungsformen aufweisen. Beispiele für die Ausführungsform des Wärmetauschers sind etwa Rohrbündelwärmetauscher, Spiralwärmetauscher oder Plattenwärmetauscher. Bevorzugt wird im Rahmen der vorliegenden Erfindung ein Wärmetauscher eingesetzt, der als Rohrbündelwärmetauscher ausgestaltet ist.

Gemäß einer insbesondere bevorzugten Ausführungsform wird die beim Entspannen des flüssigen Alkens erzeugte Kälteleistung im erfindungsgemäßen Verfahren selbst eingesetzt. Dadurch wird ein integriertes Verfahren realisiert. Der Einsatz dieser Kälteleistung kann hierbei etwa, wie oben beschrieben, über ein Kältemittel erfolgen, das im Rahmen der Entspannung des Stroms S1 abgekühlt wird und das in mindestens einem weiteren Teil des erfindungsgemäßen Verfahrens dann verwendet werden kann, um beispielsweise in einem oder mehreren Wärmetauschern beispielsweise einen Eduktstrom oder einen Produktstrom abzukühlen.

Gemäß einer besonders bevorzugten Ausführungsform des erfindungsgemäßen Verfahrens wird die durch die Entspannung des Stroms S1 in dem mindestens einen Wärmetauscher erzeugte Kälteleistung direkt genutzt, wobei unter dem Begriff "direkt" in diesem Zusammenhang im Rahmen der vorliegenden Erfindung jede Verfahrensführung verstanden wird, bei der zur Abkühlung der Umweg über ein Kältemittel nicht beschritten wird.

Insbesondere bevorzugt sind hierbei im Rahmen der vorliegenden Erfindung Ausführungsformen, bei denen einem oder mehreren im erfindungsgemäßen Verfahren anfallenden Strömen in dem mindestens einen Wärmetauscher, in dem der Strom S 1 entspannt wird, Wärme entzogen wird.

Demgemäß betrifft die vorliegende Erfindung auch ein Verfahren, wie oben beschrieben, das dadurch gekennzeichnet ist, dass die durch die Entspannung gemäß (ii) erzeugte Kälteleistung in mindestens einem Wärmetauscher mindestens teilweise mindestens einem Teilprozess der Alkenoxidherstellung zugeführt wird.

Als Teilprozess der Alkenoxidherstellung sind sämtliche Verfahrensschritte möglich, in denen die gemäß (ii) durch Entspannungsverdampfen erzeugte Kälteleistung zu einem Abkühlprozess eingesetzt werden kann. Hierbei ist es beispielsweise denkbar, die gemäß (ii) erzeugte Kälteleistung zusätzlich zu einer auf anderem Weg bereitgestellten Kälteleistung einzusetzen. Ebenso kann die gemäß (ii) erzeugte Kälteleistung allein verwendet werden.

Gemäß einer beispielsweise besonders bevorzugten Ausführungsform des erfindungsgemäßen Verfahrens wird die gemäß (ii) erzeugte Kälteleistung dazu verwendet, bei der Kondensation von Brüden am Kopf einer oder mehrerer Destillationskolonnen des erfindungsgemäßen Verfahrens die dazu notwendige Kälteleistung zumindest teilweise zur Verfügung zu stellen. Insbesondere ist dies bei der Herstellung von Propylenoxid der Fall, und hierbei wiederum bevorzugt bei einer destillativen Aufarbeitung eines Gemisches, das Propylenoxid enthält und bei der ein Brüden erhalten wird, der Propylenoxid enthält.

Dies rührt daher, dass es sich bei Propylenoxid um eine hochreaktive Verbindung mit niedrigem Siedepunkt, d.h. einem Siedepunkt von 35 °C bei Umgebungsdruck, handelt. Demzufolge muss die destillative Aufarbeitung unter milden Bedingungen erfolgen, so dass zur Kondensation der Brüden am Kopf der Destillationskolonne Kälteträger eingesetzt werden müssen.

Nach dem Stand der Technik wird eine solche Kälteleistung beispielsweise unter Verwendung von Kompressions-, Dampfstrahl- oder Absorptionskältemaschinen erzeugt. Kaltdampfmaschinen arbeiten mit geschlossenen Kreisprozessen, die die Verdampfungswärme eines verflüssigten Mediums zur Kälteerzeugung ausnutzen. Die Verwendung solcher Kaltdampfmaschinen hat zur Folge, dass zur Bereitstellung der Kälteleistung in zusätzliche Apparate wie beispielsweise Pumpen, Verdichter oder Verdampfer investiert und diese betrieben werden müssen.

Die wie oben beschriebene direkte Nutzung der Verdampfungsenthalpie des im erfindungsgemäß eingesetzten Alkens, insbesondere bevorzugt des Propens, stellt demgegenüber ein Verfahren zur Verfügung, bei dem teuere Verdampfungskälte auf niedrigem Temperaturniveau gewonnen werden kann. Selbstverständlich, wie oben beschrieben, kann hierbei die Übertragung der Kälteleistung über mindestens ein Kältemittel erfolgen, doch ist es bevorzugt, den zu entspannenden Strom S1 in einem Kompartiment eines Wärmetauschers, der Heizseite, zu entspannen, wobei in einem anderen Kompartiment dieses Wärmetauschers, der Kühlseite, der Brüden, zu dessen Kondensation Kälteleistung aufgebracht werden soll, abgekühlt wird.

Wie oben beschrieben, kann im erfindungsgemäßen Verfahren der zu entspannende Strom S1 gemäß der zur Kondensation des Brüden benötigten Kälteleistung in zwei oder mehr Teilströme geteilt werden, wobei jeder der Teilströme in jeweils einen Wärmetauscher hinein entspannt und vollständig verdampft und der Brüden in demjenigen Wärmetauscher kondensiert wird, in dem die benötigte Kälteleistung zur Verfügung gestellt wird.

Bei der wie oben beschriebenen destillativen Aufarbeitung eines Alkenoxid, bevorzugt Propylenoxid enthaltenden Gemisches, bei der ein Brüden anfällt, der im Wesentlichen Alkenoxid, bevorzugt Propylenoxid, enthält, wird besonders bevorzugt ein Gemisch destillativ aufgearbeitet, das neben Alkenoxid, bevorzugt Propylenoxid, im wesentlichen das gemäß (v) eingesetzte Lösungsmittel, bevorzugt Methanol, sowie gegebenenfalls im Vergleich zu Alkyenoxid, bevorzugt Propylenoxid, höher siedende Verbindungen enthält.

Demgemäß beschreibt die vorliegende Erfindung auch ein Verfahren, wie oben beschrieben, das dadurch gekennzeichnet ist, dass der mindestens eine Teilprozess der Alkenoxidherstellung, dem die durch die Entspannung gemäß (ii) aufgenommene Wärme in mindestens einem Wärmetauscher mindestens teilweise entzogen wird, die Kondensation eines Brüden ist.

Insbesondere betrifft die vorliegende Erfindung daher auch ein Verfahren, wie oben beschrieben, das dadurch gekennzeichnet ist , dass der Teilprozess der Alkenoxidherstellung die Kondensation eines Brüden ist, der im Wesentlichen Alkenoxid enthält und der bei der destillativen Abtrennung von Alkenoxid aus einem Gemisch (G1), enthaltend Alkenoxid und mindestens ein Lösungsmittel, erhalten wird.

Grundsätzlich ist es im Rahmen der vorliegenden Erfindung möglich, sämtliche Gemische (G1) aufzuarbeiten, die Alkenoxid und mindestens ein Lösungsmittel enthalten unter der Maßgabe, dass bei der destillativen Aufarbeitung der wie oben beschriebene Brüden resultiert. Insbesondere kann ein Gemisch destillativ unter Erhalt des genannten Brüden aufgearbeitet werden, das gemäß Schritt (v) des erfindungsgemäßen Verfahrens erhalten wird.

In einer weiter bevorzugten Ausführungsform des Schrittes (v) des erfindungsgemäßen Verfahrens, der diesbezüglich im Folgenden mit (v') bezeichnet wird, wird ein Gemisch erhalten, das neben dem Alkenoxid und dem mindestens einen Lösungsmittel auch nicht umgesetztes Alken enthält. In diesem Fall ist es besonders bevorzugt, dieses Gemisch vor der destillativen Aufarbeitung unter Erhalt des oben genannten Brüden mindestens einer weiteren Aufarbeitungsstufe zuzuführen, in der das nicht umgesetzte Alken abgetrennt wird.

Gemäß einer besonders bevorzugten Ausführungsform des erfindungsgemäßen Verfahrens wird das Gemisch (G1) demgemäß dadurch erhalten, dass ein Alken mit einem Hydroperoxid in Anwesenheit mindestens eines Lösungsmittels und mindestens eines Katalysators umgesetzt wird, wobei ein Gemisch erhalten wird, das das Alkenoxid sowie nicht umgesetztes Alken und das mindestens eine Lösungsmittel enthält. Dieses Gemisch wird im Folgenden mit (G0) bezeichnet. Aus diesem Gemisch (G0) wird vor der destillativen Abtrennung, aus der der Brüden, der im wesentlichen Alkenoxid enthält, resultiert, in mindestens einem vorgelagerten Schritt das nicht umgesetzte Alken abgetrennt und aus dieser Alkenabtrennung das Gemisch (G1) erhalten.

Demgemäß betrifft die vorliegende Erfindung auch ein Verfahren, wie oben beschrieben, das dadurch gekennzeichnet ist, dass das Gemisch (G1) aus einem Verfahren erhalten wird, das die folgenden Schritte (v') und (vi) umfasst:
(v') Umsetzung des gemäß (ii) erhaltenen Alkens mit einem Hydroperoxid in Anwesenheit mindestens eines Lösungsmittels und mindestens eines Katalysators unter Erhalt eines Gemischs G(0), enthaltend Alkenoxid, nicht umgesetztes Alken und das mindestens eine Lösungsmittel;
(vi) Abtrennung des nicht umgesetzten Alkens aus dem Gemisch (G0) unter Erhalt eines Gemisches (G1), enthaltend Alkenoxid und das mindestens eine Lösungsmittel.

Die Umsetzung des Alkens, bevorzugt Propen, gemäß (v') in Anwesenheit des mindestens einen Lösungsmittels, bevorzugt Methanol, und des mindestens einen Katalysators, bevorzugt des oben beschriebenen Titansilikalitkatalysators, mittels des Hydroperoxids, bevorzugt Wasserstoffperoxid, kann in einer, zwei oder auch mehr Stufen durchgeführt werden, wobei die Umsetzung besonders bevorzugt in zwei Stufen durchgeführt wird.

Bezogen auf die oben genannten bevorzugt eingesetzten Verbindungen findet eine zweistufige Umsetzung beispielsweise folgendermaßen statt:
(a) Umsetzung des Wasserstoffperoxids mit Propen unter Erhalt einer Mischung, umfassend Propylenoxid und nicht-umgesetztes Wasserstoffperoxid;
(b) Abtrennung des nicht-umgesetzten Wasserstoffperoxids aus der aus Stufe (a) resultierenden Mischung;
(c) Umsetzung des abgetrennten Wasserstoffperoxids aus Stufe (b) mit Propen.

Demgemäß findet die Umsetzung von Propen mit Wasserstoffperoxid, wie dargestellt, in zwei Stufen (a) und (c) statt, wobei eine Abtrennstufe (b) zwischengeschaltet ist.

Die Abtrennung des Wasserstoffperoxids in der Abtrennstufe (b) kann im Rahmen der vorliegenden Erfindung nach allen gängigen Abtrennmethoden gemäß Stand der Technik durchgeführt werden.

Vorzugsweise erfolgt die Abtrennung des Wasserstoffperoxids destillativ. Je nach den Anforderungen des Verfahrens ist dabei eine Abtrennung in einer oder mehreren Destillationskolonnen möglich. Vorzugsweise wird im Rahmen einer Abtrennstufe eine Destillationskolonne eingesetzt.

Die Umsetzung von Propen mit Wasserstoffperoxid findet im erfindungsgemäßen Verfahren in einem dafür geeigneten Reaktor statt. Als Edukte der Umsetzung werden Propen, Wasserstoffperoxid und Methanol eingesetzt. Die Edukte können im Rahmen des Verfahrens einzeln oder vorzugsweise vor dem Zufluss in den Reaktor zu einem Strom vereinigt dem Reaktor zugeführt werden. Bevorzugt wird im erfindungsgemäßen Verfahren ein Strom dem Reaktor zugeleitet, welcher aus der Kombination der Edukte besteht. Dabei ist ein Strom bevorzugt, bei dem die Konzentrationen der einzelnen Edukte des Stroms so gewählt sind, dass der Strom flüssig und einphasig ist.

In einer weiteren bevorzugten Ausführungsform ist es möglich, die Umsetzung in den Stufen (a) und (c) in zwei getrennten Reaktoren durchzuführen.

Als Reaktoren können selbstverständlich alle denkbaren, für die jeweilige Reaktion am besten geeigneten Reaktoren eingesetzt werden. Dabei ist der Begriff "ein Reaktor" nicht auf einen einzigen Behälter beschränkt. Vielmehr ist es auch möglich, als Reaktor eine Rührkesselkaskade einzusetzen.

Bevorzugt werden als Reaktoren Festbettreaktoren verwendet. Weiter bevorzugt werden als Festbettreaktoren Festbettrohrreaktoren eingesetzt.

Insbesondere bevorzugt werden bei den Umsetzungen in den Stufen (a) und (c) in zwei getrennten Reaktoren ein isothermer Festbettrohrreaktor und ein adiabatischer Festbettreaktor eingesetzt. Bevorzugt werden der isotherme Festbettrohrreaktor in der Stufe (a) und der adiabatische Festbettreaktor in der Stufe (b) eingesetzt.

Nach der Umsetzung des Alkens, bevorzugt Propen, mit Hydroperoxid, bevorzugt Wasserstoffperoxid, wobei ein Gemisch (G0) erhalten wird, wird weiter bevorzugt aus diesem Gemisch (G0), enthaltend Alkenoxid, bevorzugt Propylenoxid, nicht umgesetztes Alken, bevorzugt Propen, und Lösungsmittel, bevorzugt Methanol, nicht umgesetztes Alken, bevorzugt Propen abgetrennt.

Bevorzugt erfolgt die Abtrennung des nicht-umgesetzten Alkens aus (G0) destillativ. Dabei ist die Anzahl der eingesetzten Kolonnen im Wesentlichen beliebig. Bevorzugt wird eine Kolonne verwendet. Diese Kolonne weist im Allgemeinen mindestens 5, bevorzugt mindestens 10 und weiter bevorzugt mindestens 15 theoretische Böden auf. Die Destillation in dieser Kolonne wird bevorzugt bei Drücken im Bereich von 0,5 bis 25 bar weiter, bevorzugt von 0,7 bis 5 bar und besonders bevorzugt im Bereich von 0,9 bis 1,5 bar durchgeführt.

Bei der im bevorzugten Fall erfolgenden Abtrennung des nicht-umgesetzten Propens tritt unter Umständen das Problem auf, dass beim Abtrennen des Propens als Leichtsiederfraktion sich in dieser Leichtsiederfraktion Sauerstoff in einer Konzentration ansammeln kann, die die Leichtsiederfraktion zu einem zündfähigen Gemisch macht. Dadurch kann ein ernstes Sicherheitsrisiko entstehen, wenn Propen wiederum destillativ von der Leichtsiederfraktion abgetrennt wird, was dann bevorzugt der Fall ist, wenn das Propen, wie gemäß einer Ausführungsform des erfindungsgemäßen Verfahrens bevorzugt, in das Verfahren als Edukt rückgeführt werden soll.

Dieses Problem kann beispielsweise dadurch gelöst werden, dass Propen destillativ aus dem Leichtsiedergemisch entfernt wird und im oberen Teil der dafür verwendeten Trenneinrichtung ein inerter Stoff mit einem Siedepunkt, der niedriger als der des Propens ist, bevorzugt Stickstoff oder auch beispielsweise Methan, in einer solchen Menge zuzugeben wird, dass der Sauerstoff bis zu einer Konzentration verdünnt ist, bei der das Gemisch nicht mehr zündfähig ist. Dieses Verfahren ist beispielsweise in der EP-B 0 719 768 beschrieben. Bevorzugt wird das Problem jedoch dadurch gelöst, dass ein Verfahren zur Aufarbeitung eines Gemisches, umfassend Propen und Sauerstoff, angewendet wird, in dem Sauerstoff nichtdestillativ aus dem Gemisch unter Erhalt eines weiteren Gemisches entfernt wird und aus dem weiteren Gemisch das Propen destillativ abgetrennt wird. Dieses Verfahren ist in der DE-A 100 01 401.1 beschrieben, die diesbezüglich vollumfänglich durch Bezugnahme in den Kontext der vorliegenden Anmeldung einbezogen wird.

Im Rahmen dieses Verfahrens wird aus einem Gemisch (G1), das Alken und Sauerstoff enthält, der Sauerstoff beispielsweise bevorzugt durch Verbrennen oder durch Reaktion des Sauerstoffs mit einer geeigneten chemischen Verbindung abgetrennt. Die Verbrennung wird dabei bevorzugt in Anwesenheit eines geeigneten Katalysators, wie beispielsweise eines geträgerten Edelmetallkatalysators durchgeführt, wobei Temperaturen von bevorzugt 280 - 580 °C angewandt werden. Als geeignete chemische Verbindung, mit der der Sauerstoff abgetrennt wird, ist unter anderem bevorzugt ein Alkan zu nennen, das wiederum bevorzugt bereits in (G1) enthalten ist. Als bevorzugte Reaktion des Alkans, beispielsweise Propans mit Sauerstoff ist eine Oxydehydrogenierung zu nennen, die weiter bevorzugt in Anwesenheit eines geeigneten Katalysators durchgeführt wird, wie er beispielsweise in M. Xu, React. Kinet. Catal. Lett. 57 (1996), S. 3-11 oder B. Delmon. Stud. Surf. Sci. Catal. 110 (1997), S. 43-59 beschrieben ist.

Im Rahmen einer bevorzugten Ausführungsform des erfindungsgemäßen Verfahrens enthält das Gemisch (G1) Propylenoxid in einem Anteil im Bereich von 5 bis 15 Gew.-%, bevorzugt von 6 bis 12 Gew.-% und besonders bevorzugt von 8 bis 10,5 Gew.-%, und Methanol in einem Anteil im Bereich von 55 bis 85 Gew.-%, bevorzugt von 60 bis 80 Gew.-% und besonders bevorzugt von 65 bis 75 Gew.-%.

Gemäß einer weiteren bevorzugten Ausführungsform des erfindungsgemäßen Verfahrens wird der wie oben beschriebene Brüdenstrom, im Wesentlichen enthaltend Alkenoxid, komprimiert und anschließend kondensiert. Die bei dieser Kondensation anfallende Wärme kann gemäß einer bevorzugten Ausführungsform mindestens zum Teil in das erfindungsgemäße Verfahren, bevorzugt in mindestens einen Verdampfer einer Destillationskolonne und weiter bevorzugt in mindestens einen Verdampfer der Destillationskolonne, in der das Gemisch (G1) destillativ unter Erhalt eines Brüdenstroms, im wesentlichen enthaltend Alkenoxid, rückgeführt werden. Ein Teil des erhaltenen Kondensats wird gemäß einer weiter bevorzugten Ausführungsform, des erfindungsgemäßen Verfahrens im Anschluss weiter abgekühlt und als Rücklauf auf die Destillationskolonne gegeben, aus der der genannte Brüdenstrom erhalten wird. Dieser Schritt des weiteren Abkühlens des Kondensats findet bevorzugt in einem Wärmetauscher statt, in dem das Kondensat bevorzugt auf eine Temperatur im Bereich von 10 bis 30 °C und besonders bevorzugt auf eine Temperatur im Bereich von 12 bis 20 °C abgekühlt wird. Die dazu erforderliche Kälteleistung kann beispielsweise besonders bevorzugt dadurch aufgebracht werden, dass in diesem Wärmetauscher auf der Heizseite der komprimierte Alkenstrom unter mindestens teilweisem, bevorzugt vollständigem Verdampfen entspannt wird, wie dies im Rahmen der vorliegenden Erfindung oben beschrieben ist.

Demgemäß beschreibt die vorliegende Erfindung auch ein Verfahren, wie oben beschrieben, das dadurch gekennzeichnet ist, dass in mindestens einem der Wärmetauscher gemäß (ii) die Wärme mindestens teilweise einem Brüdenkondensat entzogen wird, das gemäß Schritt (cc) eines Verfahrens gewonnen wird, das mindestens folgende Schritte (aa) bis (dd) umfasst:
(aa) Destillative Abtrennung von Alkenoxid aus einem Gemisch (G1), enthaltend Alkenoxid und mindestens ein Lösungsmittel, unter Verwendung einer Destillationskolonne, wobei ein Sumpfstrom und ein Brüdenstrom, im Wesentlichen enthaltend Alkenoxid, erhalten wird;
(bb) Komprimierung des gemäß (aa) erhaltenen Brüdenstroms mittels mindestens eines Verdichters unter Erhalt eines verdichteten Brüden;
(cc) Kondensation des gemäß (bb) erhaltenen Brüden unter Rückführung mindestens eines Teils der Kondensationswärme in mindestens einen in der gemäß (aa) verwendeten Destillationskolonne eingesetzten Verdampfer;
(dd) Abkühlen mindestens eines Teils des gemäß (cc) erhaltenen Kondensats in mindestens einem Wärmetauscher auf eine Temperatur im Bereich von 12 bis 20 °C und Rückführung mindestens eines Teils des abgekühlten Kondensats als Rücklauf auf die gemäß (aa) verwendete Destillationskolonne.

Bevorzugt wird gemäß (dd) nicht das gesamte aus (cc) erhaltene Kondensat dem mindestens einen Wärmetauscher zugeführt, wobei weiter bevorzugt der Teil, der in dem mindestens einen Wärmetauscher abgekühlt wird, vollständig als Rücklauf in die gemäß (aa) verwendete Destillationskolonne rückgeführt.

Der gemäß (dd) eingesetzte Wärmetauscher kann im Wesentlichen beliebig ausgestaltet sein. Beispiele für die Ausführungsform des Wärmetauschers sind etwa Rohrbündelwärmetauscher, Spiralwärmetauscher oder Plattenwärmetauscher. Bevorzugt wird im Rahmen der vorliegenden Erfindung ein Wärmetauscher eingesetzt, der als Rohrbündelwärmetauscher ausgestaltet ist.

Erfindungsgemäß wird der aus (ii) erhaltene entspannte und verdampfte Alkenstrom als Edukt in die Umsetzung gemäß (v) eingesetzt. Dabei kann dieser Alkenstrom beispielsweise als solcher in die zur Umsetzung verwendete, mindestens eine Vorrichtung wie beispielsweise die oben gemäß den Schritten (a) und (c) beschriebenen Reaktoren, eingeführt werden.

Bevorzugt ist es im erfindungsgemäßen Verfahren, den entspannten und verdampften, gasförmigen Alkenstrom vor der Einführung in die zur Umsetzung verwendete Vorrichtung in mindestens eines der verwendeten Lösungsmittel einzulösen. Hinsichtlich des bevorzugt verwendeten Propens wird demgemäß der verdampfte, entspannte Propenstrom vor der Einführung in die mindestens eine zur Epoxidierung verwendete Vorrichtung in Methanol eingelöst.

Demgemäß betrifft die vorliegende Erfindung auch ein Verfahren, wie oben beschrieben, das zusätzlich folgende Schritte (iii) und (iv) umfasst:
(iü) Lösen des gemäß (ii) erhaltenen gasförmigen Alkens in mindestens einem der gemäß (v) oder (v') verwendeten Lösungsmittel unter Erhalt einer Lösung;
(iv) Einleiten der gemäß (iü) erhaltenen Lösung in die zur Umsetzung gemäß (v) oder (v') verwendete Vorrichtung.

Das Einlösen gemäß (iü) kann grundsätzlich gemäß sämtlicher geeigneter Methoden und Verfahrensführungen erfolgen. Besonders bevorzugt ist es, das Alken über einen Injektor anzusaugen und in dem Lösungsmittel einzulösen, wobei der Injektor ganz besonders bevorzugt mit diesem Lösungsmittel als Treibmittel betrieben wird. Gemäß der oben beschriebenen bevorzugten Ausführungsform wird gemäß (iii) das aus (ii) erhaltene gasförmige Propen in Methanol unter Verwendung eines mit Methanol als Treibmittel betriebenen Injektors eingelöst und die erhaltene Lösung gemäß (iv) in die mindestens eine zur Umsetzung gemäß (v) oder (v') verwendete Vorrichtung eingeleitet.

Wird, wie oben beschrieben, der Strom S1 in zwei oder mehr Teilströme geteilt und jeder der Teilströme in jeweils einen Wärmetauscher hinein entspannt und vollständig verdampft, so werden gemäß einer bevorzugten Ausführungsform des erfindungsgemäßen Verfahrens die erhaltenen gasförmigen Teilströme nach dem Verlassen der Wärmetauscher vereinigt und gemeinsam in einem Injektor eingelöst.
Die beim Einlösen des Alkenoxids in das Lösungsmittel erzeugte Lösungswärme kann beispielsweise im erfindungsgemäßen oder einem anderen Verfahren genutzt werden. Besonders bevorzugt wird diese Lösungswärme über einen Wärmetaucher mittels herkömmlichen Flusswassers abgeführt. Bevorzugt erfolgt diese Abführung der Lösungswärme bei einer Temperatur im Bereich von 25 bis 45 °C.

Demgemäß betrifft die vorliegende Erfindung auch ein Verfahren, wie oben beschrieben, das dadurch gekennzeichnet ist, dass die gemäß (iii) auftretende Lösungswärme mittels Flusswassers abgeführt wird.

Durch diese spezielle Verfahrensführung des erfindungsgemäßen Verfahrens wird es ermöglicht, durch die Entspannungsverdampfung des Alkenstroms eine ansonsten nur durch teuere Maßnahmen zur Verfügung stehende Kälteleistung auf niedrigem Temperaturniveau wie beispielsweise im Bereich von 3 bis 10 °C zu gewinnen und andererseits die gemäß (iii) anfallende Lösungsenthalpie des Alkens preiswert mit Flusswasser abzuführen.

Das zum Betreiben des Injektors verwendete Lösungsmittel, insbesondere bevorzugt Methanol; wird im erfindungsgemäßen Verfahren bevorzugt im Kreis geführt. Das Lösungsmittel, in dem das entspannte Alken eingelöst wird, wird demgemäß nach einer bevorzugten Ausführungsform aus dem Gemisch (G1) abgetrennt, wobei die destillative Aufarbeitung des Gemischs (G1) bevorzugt so erfolgt, dass der Brüdenstrom, enthaltend im wesentlichen Alkenoxid, Lösungsmittel im Bereich von höchstens 500 ppm, bevorzugt von höchstens 200 ppm, weiter bevorzugt von höchstens 100 ppm, weiter bevorzugt von höchstens 50 ppm, weiter bevorzugt von höchstens 20 ppm, besonders bevorzugt von höchstens 15 ppm und insbesondere bevorzugt von höchstens 10 ppm, jeweils bezogen auf das Gesamtgewicht der abgetrennten Alkenoxidfraktion, enthält.

Die Abtrennung des Lösungsmittels, bevorzugt Methanol, aus (G1) erfolgt bevorzugt destillativ. In einem besonders bevorzugten Verfahren, in dem Propen in Methanol mit einer wässrigen Wasserstoffperoxidlösung umgesetzt wird, fällt bei dieser destillativen Abtrennung beispielsweise ein Sumpfstrom an, der Methanol und Wasser enthält. Gemäß einer weiter bevorzugten Ausführungsform wird aus diesem Sumpfstrom, umfassend Methanol und Wasser, Wasser abgetrennt und ein aus dieser Abtrennung resultierender Abstrom, umfassend Methanol und Wasser, in das Verfahren zurückgeführt, wobei dieser Abstrom Wasser im Bereich von mindestens 3 Gew.-%, bevorzugt im Bereich von 3 bis 10 Gew.-%, bezogen auf das Gesamtgewicht des Abstroms, enthält. Weiter bevorzugt enthält im Rahmen der beschriebenen Umsetzung von Propen der Sumpfstrom neben Methanol und Wasser auch Methylformiat. Bevorzugt umfasst dieses Verfahren daher folgenden Schritte:
- Abtrennung von Wasser aus dem Sumpfstrom unter Erhalt eines Abstroms A1, enthaltend Methanol, Methylformiat und Wasser;
- Abtrennung von Methylformiat aus dem Abstrom A1 unter Erhalt eines Abstroms A2, enthaltend Methanol und Wasser;
- Rückführung des Abstroms A2 in das Verfahren, wobei A2 Wasser in den oben genannten Grenzen enthält.

Besonders bevorzugt wird A2 als Lösungsmittel in bevorzugt (a) und/oder (c), bevorzugt (a) rückgeführt, wobei beispielsweise das Alken erfindungsgemäß in A2 zumindest teilweise eingelöst werden kann.

Daher betrifft die vorliegende Erfindung auch ein Verfahren, wie oben beschrieben, das dadurch gekennzeichnet ist, dass das gemäß (iii) verwendete Lösungsmittel im Kreis geführt wird.

Im folgenden Beispiel wird die vorliegende Erfindung näher erläutert.

### Beispiel

Gemäß dem in WO 00/07965 beschriebenen Verfahren wird Propylenoxid ausgehend von Propen durch Umsetzung mit Wasserstoffperoxid in Methanol als Lösungsmittel unter Verwendung eines TS-1-Katalysators hergestellt.

Durch die Entspannung und vollständige Verdampfung von 1 t flüssigen Propens von 30 bar und einer Temperatur von 25 °C auf einen Druck von 6,3 bar wurde eine Kälteleistung von 84 kWh/t (Propen) bei einer Temperatur von 3,5 °C erhalten. Die anschließende Einlösung des gasförmigen Propens in Methanol erfolgte mittels eines Injektors, wie er beispielsweise von der Firma GEA Jet Pumps geliefert wird. Zum Einlösen von 1 t Propen wurden 7 t Methanol, das bei der Reaktion als Lösungsmittel eingesetzt wurde, als Treibmittel verwendet.

Das propenhaltige Methanol, dem die entsprechende Lösungswärme bei 35 °C mit Flusswasser abgeführt wurde, wurde dann zur Umsetzung von Propen mit Wasserstoffperoxid einem entsprechenden Oxidationsreaktor zugeführt.

Ein Teil der anfallenden Kälteleistung von 84 kWh/t (Propen) wurde im Verfahren zur Herstellung von Propylenoxid zur Kondensation des Brüden der Propylenoxid-Destillation eingesetzt, bei der Propylenoxid von restlichem Lösungsmittel getrennt wurde.

## Patentansprüche

1. Verfahren zur Herstellung eines Alkenoxids, umfassend mindestens die Schritte (i), (ii) und (v):
(i) Bereitstellen eines Stroms S1, enthaltend komprimiertes, flüssiges Alken;
(ii) Entspannen mindestens eines Teils des Stroms S1 unter Aufnahme von Wärme und unter mindestens teilweisem Verdampfen des flüssigen Alkens;
(v) Umsetzung des gemäß (ii) erhaltenen Alkens mit einem Hydroperoxid in Anwesenheit mindestens eines Lösungsmittels und mindestens eines Katalysators unter Erhalt eines Gemischs, enthaltend Alkenoxid und das mindestens eine Lösungsmittel.

2. Verfahren nach Anspruch 1, **dadurch gekennzeichnet, dass** das Alken Propen, das Hydroperoxid Wasserstoffperoxid, der verwendete Katalysator ein Titansilikalit-Katalysator und das Lösungsmittel Methanol ist.

3. Verfahren nach Anspruch 2, **dadurch gekennzeichnet, dass** der Strom S1 gemäß (i) flüssiges Propen bei einem Druck im Bereich von 20 bis 35 bar und einer Temperatur im Bereich von 5 bis 30 °C enthält.

4. Verfahren nach Anspruch 2 oder 3, **dadurch gekennzeichnet, dass** der mindestens eine Teil des Stroms S1 auf einen Druck im Bereich von 4 bis 10 bar entspannt wird.

5. Verfahren nach einem der Ansprüche 1 bis 4, **dadurch gekennzeichnet, dass** die durch die Entspannung gemäß (ii) erzeugte Kälteleistung in mindestens einem Wärmetauscher mindestens teilweise mindestens einem Teilprozess der Alkenoxidherstellung zugeführt wird.

6. Verfahren nach Anspruch 5, **dadurch gekennzeichnet, dass** der Teilprozess der Alkenoxidherstellung die Kondensation eines Brüden ist, der im Wesentlichen Alkenoxid enthält und der bei der destillativen Abtrennung von Alkenoxid aus einem Gemisch (G1), enthaltend Alkenoxid und mindestens ein Lösungsmittel, erhalten wird.

7. Verfahren nach Anspruch 6, **dadurch gekennzeichnet, dass** das Gemisch (G1) aus einem Verfahren erhalten wird, das die folgenden Schritte (v') und (vi) umfasst:
(v') Umsetzung des gemäß (ii) erhaltenen Alkens mit einem Hydroperoxid in Anwesenheit mindestens eines Lösungsmittels und mindestens eines Katalysators unter Erhalt eines Gemischs (G0), enthaltend Alkenoxid, nicht umgesetztes Alken und das mindestens eine Lösungsmittel;
(vi) Abtrennung des nicht umgesetzten Alkens aus dem Gemisch (G0) unter Erhalt eines Gemisches (G1), enthaltend Alkenoxid und das mindestens eine Lösungsmittel.

8. Verfahren nach einem der Ansprüche 1 bis 7, zusätzlich umfassend folgende Schritte (iii) und (iv):
(iii) Lösen des gemäß (ii) erhaltenen gasförmigen Alkens in mindestens einem der gemäß (v) oder (v') verwendeten Lösungsmittel unter Erhalt einer Lösung;
(iv) Einleiten der gemäß (iii) erhaltenen Lösung in die zur Umsetzung gemäß (v) oder (v') verwendete Vorrichtung.

9. Verfahren nach Anspruch 8, **dadurch gekennzeichnet, dass** die gemäß (iii) auftretende Lösungswärme mittels Flusswassers abgeführt wird.

10. Verfahren nach Anspruch 8 oder 9, **dadurch gekennzeichnet, dass** das gemäß (iü) verwendete Lösungsmittel im Kreis geführt wird.

## Claims

1. A process for preparing an alkene oxide, which comprises at least the steps (i), (ii) and (v):
(i) providing a stream S1 comprising compressed, liquid alkene;
(ii) depressurizing at least part of the stream S 1 with absorption of heat and with at least partial vaporization of the liquid alkene;
(v) reacting the alkene obtained in (ii) with a hydroperoxide in the presence of at least one solvent and at least one catalyst to give a mixture comprising alkene oxide and the solvent or solvents.

2. The process according to claim 1, wherein the alkene is propene, the hydroperoxide is hydrogen peroxide, the catalyst used is a titanium silicalite catalyst and the solvent is methanol.

3. The process according to claim 2, wherein the stream S1 in (i) comprises liquid propene at a pressure in the range from 20 to 35 bar and a temperature in the range from 5 to 30°C.

4. The process according to claim 2 or 3, wherein the part or parts of stream S1 is/are depressurized to a pressure in the range from 4 to 10 bar.

5. The process according to any of claims 1 to 4, wherein at least part of the refrigeration effect produced by the depressurization in (ii) is transferred in at least one heat exchanger to at least one suboperation of the alkene oxide production process.

6. The process according to claim 5, wherein the suboperation of the alkene oxide production process is the condensation of a vapor which comprises essentially alkene oxide and is obtained in the separation of alkene oxide from a mixture (M1) comprising alkene oxide and at least one solvent by distillation.

7. The process according to claim 6, wherein the mixture (M1) is obtained from a process comprising the steps (v') and (vi):
(v') reacting the alkene obtained in (ii) with a hydroperoxide in the presence of at least one solvent and at least one catalyst to give a mixture (M0) comprising alkene oxide, unreacted alkene and the solvent or solvents;
(vi) separating the unreacted alkene from the mixture (M0) to give a mixture (M1) comprising alkene oxide and the solvent or solvents.

8. The process according to any of claims 1 to 7 which further comprises the steps (iii) and (iv):
(iii) dissolving the gaseous alkene obtained in (ii) in at least one of the solvents used in (v) or (v') to give a solution;
(iv) introducing the solution obtained in (iii) into the apparatus used for the reaction of (v) or (v').

9. The process according to claim 8, wherein the heat of solution evolved in (iii) is removed by means of river water.

10. The process according to claim 8 or 9, wherein the solvent used in (iii) is circulated.

## Revendications

1. Procédé pour fabriquer un oxyde d'alcène, comprenant au moins les étapes (i), (ii) et (v) consistant à :
(i) préparer un courant S1 contenant un alcène liquide sous pression ;
(ii) effectuer la détente d'au moins une partie du courant S1 tout en absorbant de la chaleur et en vaporisant au moins partiellement l'alcène liquide ;
(v) faire réagir l'alcène obtenu à l'étape (ii) avec un hydroperoxyde, en présence d'au moins un solvant et d'au moins un catalyseur, pour obtenir un mélange contenant un oxyde d'alcène et ledit au moins un solvant.

2. Procédé selon la revendication 1, **caractérisé en ce que** l'alcène est le propène, l'hydroperoxyde est le peroxyde d'hydrogène, le catalyseur utilisé est un catalyseur à base de silicalite de titane et le solvant est le méthanol.

3. Procédé selon la revendication 2, **caractérisé en ce que** le courant S1 selon l'étape (i) contient du propène liquide à une pression dans la plage de 20 à 35 bar et à une température dans la plage de 5 à 30°C.

4. Procédé selon la revendication 2 ou 3, **caractérisé en ce que** ladite au moins une partie du courant S1 est détendue à une pression dans la plage de 4 à 10 bar.

5. Procédé selon l'une quelconque des revendications 1 à 4, **caractérisé en ce que** la puissance frigorifique générée par la détente selon l'étape (ii) est acheminée au moins en partie dans au moins un échangeur de chaleur à une étape du processus de fabrication de l'oxyde d'alcène.

6. Procédé selon la revendication 5, **caractérisé en ce que** l'étape du processus de fabrication de l'oxyde d'alcène est la condensation de fumées, qui contiennent essentiellement l'oxyde d'alcène et qui sont obtenues lors de la séparation par distillation de l'oxyde d'alcène à partir d'un mélange (G1) contenant l'oxyde d'alcène et au moins un solvant.

7. Procédé selon la revendication 6, **caractérisé en ce que** l'on obtient le mélange (G1) à partir d'un procédé qui comprend les étapes (v') et (vi) suivantes consistant à :
(v') faire réagir l'alcène obtenu selon l'étape (ii) avec un hydroperoxyde en présence d'au moins un solvant et d'au moins un catalyseur, pour obtenir un mélange (G0) contenant de l'oxyde d'alcène, de l'alcène n'ayant pas réagi et ledit au moins un solvant ;
(vi) séparer l'alcène n'ayant pas réagi du mélange (G0) pour obtenir un mélange (G1) contenant de l'oxyde d'alcène et ledit au moins un solvant.

8. Procédé selon l'une quelconque des revendications 1 à 7, comprenant en outre les étapes (iii) et (iv) suivantes consistant à :
(iii) dissoudre l'alcène obtenu sous forme gazeuse à l'étape (ii) dans au moins un des solvants utilisés selon les étapes (v) ou (v') pour obtenir une solution ;
(iv) introduire la solution obtenue à l'étape (iii) dans le dispositif utilisé pour la réaction selon l'étape (v) ou (v').

9. Procédé selon la revendication 8, **caractérisé en ce que** la chaleur de dissolution produite à l'étape (iii) est dissipée avec de l'eau de rivière.

10. Procédé selon la revendication 8 ou 9, **caractérisé en ce que** le solvant employé à l'étape (iii) est recyclé.
